(19) Europäisches Patentamt / European Patent Office / Office européen des brevets

(11) **EP 3 235 437 A1**

(12) **EUROPEAN PATENT APPLICATION**
published in accordance with Art. 153(4) EPC

(43) Date of publication:
25.10.2017 Bulletin 2017/43

(51) Int Cl.:
*A61B 8/08* (2006.01)

(21) Application number: 15869637.7

(22) Date of filing: 09.10.2015

(86) International application number:
PCT/JP2015/078840

(87) International publication number:
WO 2016/098429 (23.06.2016 Gazette 2016/25)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 19.12.2014 JP 2014257976

(71) Applicant: OLYMPUS CORPORATION
Hachioji-shi
Tokyo 192-8507 (JP)

(72) Inventor: MISONO, Kazuhiro
Tokyo 192-8507 (JP)

(74) Representative: Gunzelmann, Rainer
Wuesthoff & Wuesthoff
Patentanwälte PartG mbB
Schweigerstraße 2
81541 München (DE)

(54) **ULTRASONIC OBSERVATION DEVICE**

(57) An ultrasound observation apparatus according to the present invention is configured to generate a combined image by combining a plurality of ultrasound images generated based on an echo signal being an electrical signal converted from an ultrasound echo being ultrasound that is transmitted to an observation target and reflected from the observation target. The ultrasound observation apparatus includes a frame memory, a frame memory correlation unit, a frame memory position correction unit, and a weighting and adding unit. The frame memory stores the plurality of ultrasound images in chronological order. The frame memory correlation unit detects a moving amount and a moving direction, as three-dimensional correlation, of the ultrasound image of a past frame with respect to the ultrasound image of a latest frame, among the plurality of ultrasound images stored in the frame memory. The frame memory position correction unit moves a relative position of the ultrasound image of the past frame based on the three-dimensional moving amount and moving direction. The weighting and adding unit performs weighting processing on the ultrasound image of the past frame based on the three-dimensional moving amount.

FIG.1

EP 3 235 437 A1

## Description

Field

The present invention relates to an ultrasound observation apparatus for observing a tissue as an observation target using ultrasound.

Background

In order to observe a characteristic of a living tissue or of a material, as an observation target, ultrasound is employed in some cases. Specifically, ultrasound is transmitted onto the observation target and reflected, by the observation target, as an ultrasound echo. By performing predetermined signal processing on this ultrasound echo, information on characteristics of the observation target is obtained.

In observation of the tissue as the observation target using ultrasound, an ultrasound image is generated based on the ultrasound echo and the observation target is observed with the generated ultrasound image being displayed. As a technique to reduce noise and blur on an ultrasound image, there is a known ultrasound observation apparatus that separates an input image into a signal component image and a noise component image, performs frame combining processing on the noise component image, and thereafter, combines the signal component image with the noise component image that has undergone the frame combining processing (for example, refer to Patent Literature 1).

Citation List

Patent Literature

Patent Literature 1: WO 2010/125789 A1

Summary

Technical Problem

Noise reduction can be more effective with more frames to be combined. In contrast, blur reduction is enhanced more with less frames to be combined. In this manner, noise reduction and blur reduction have a trade-off relationship. The combining processing disclosed in Patent Literature 1 assumes that imaging is performed on a same imaging plane, and thus, misalignment is corrected two-dimensionally. Therefore, in a case where imaging performed on different imaging planes causes misalignment of a position and an angle of an object between the frames, misalignment correction accuracy is deteriorated, causing failure in appropriate position correction. As a result, it is not possible to obtain an ultrasound image achieving both noise reduction and blur reduction.

The present invention has been made in view of the foregoing, and an object of the present invention is to provide an ultrasound observation apparatus capable of achieving both noise reduction and blur reduction in an ultrasound image.

Solution to Problem

In order to solve the above described problems and achieve the object, an ultrasound observation apparatus according to the invention is configured to generate a combined image by combining a plurality of ultrasound images generated based on an echo signal, the echo signal being obtained by converting an ultrasound echo into an electrical signal, the ultrasound echo being obtained by transmitting ultrasound to an observation target and by reflecting the ultrasound from the observation target. The ultrasound observation apparatus includes: a frame memory configured to store the plurality of ultrasound images in chronological order; a frame memory correlation unit configured to detect a moving amount and a moving direction as a three-dimensional correlation between an ultrasound image of a past frame and an ultrasound image of a latest frame, among the plurality of ultrasound images stored in the frame memory; a frame memory position correction unit configured to move a relative position of the ultrasound image of the past frame with respect to the ultrasound image of the latest frame based on the three-dimensional moving amount and moving direction detected by the frame memory correlation unit; and a weighting and adding unit configured to perform weighting processing on the ultrasound image of the past frame based on the three-dimensional moving amount detected by the frame memory correlation unit.

In the ultrasound observation apparatus according to the above-described invention, the weighting and adding unit is configured to perform the weighting processing on the ultrasound image of the past frame based on a weighting amount correlated with the moving amount detected by the frame memory correlation unit.

In the ultrasound observation apparatus according to the above-described invention, the frame memory correlation unit is configured to divide the ultrasound image of the past frame to generate divided regions, and to detect the three-dimensional moving amount and moving direction for each of the divided regions.

In the ultrasound observation apparatus according to the above-described invention, based on a plurality of three-dimensional moving amounts and moving directions each of which is detected for each of the divided regions, the frame memory correlation unit is configured to calculate the moving amount of whole of the ultrasound image of the past frame with respect to the ultrasound image of the latest frame.

In the ultrasound observation apparatus according to the above-described invention, the frame memory correlation unit is configured to: calculate a representative moving amount of the ultrasound image of a current frame based on a plurality of three-dimensional moving amounts each of which is detected for each of the divided regions; compare the representative moving amount with a prescribed value; and exclude the ultrasound image of the current frame from combining according to a result of comparison.

In the ultrasound observation apparatus according to the above-described invention, based on a plurality of three-dimensional moving amounts and moving directions each of which is detected for each of the divided regions, the frame memory correlation unit is configured to identify a moving state of whole of the ultrasound image of the past frame with respect to the ultrasound image of the latest frame.

In the ultrasound observation apparatus according to the above-described invention, the frame memory correlation unit is configured to: identify a moving state of the ultrasound image of a current frame based on a plurality of three-dimensional moving amounts each of which is detected for each of the divided regions; and exclude the ultrasound image of the current frame from combining, or reduce the weighting amount if the moving state differs from a moving state of the ultrasound image of the latest frame.

In the ultrasound observation apparatus according to the above-described invention, the moving state is a three-dimensional moving state of an object in the ultrasound images.

In the ultrasound observation apparatus according to the above-described invention, the frame memory correlation unit is configured to identify the moving state by comparing a dispersion value of the moving amounts with a predetermined value.

In the ultrasound observation apparatus according to the above-described invention, the frame memory correlation unit is configured to detect a feature point from the ultrasound image of the latest frame, and to detect the correlation between the ultrasound image of the past frame and the ultrasound image of the latest frame by using the feature point.

Advantageous Effects of Invention

According to the present invention, it is possible to achieve both noise reduction and blur reduction in an ultrasound image.

Brief Description of Drawings

FIG. 1 is a block diagram illustrating a configuration of an ultrasound observation system including an ultrasound observation apparatus according to an embodiment of the present invention.
FIG. 2 is a diagram illustrating a relationship between a reception depth and an amplification factor in amplification processing performed by a beamformer of the ultrasound observation apparatus according to an embodiment of the present invention.
FIG. 3 is a diagram schematically illustrating movement of images with different imaging timings, performed by a frame memory correlation unit of the ultrasound observation apparatus according to an embodiment of the present invention.
FIG. 4 is a flowchart illustrating an outline of processing performed by the ultrasound observation apparatus according to an embodiment of the present invention.
FIG. 5 is a schematic diagram illustrating motion vector detection processing performed by the frame memory correlation unit of the ultrasound observation apparatus according to an embodiment of the present invention.
FIG. 6 is a schematic diagram illustrating motion vector detection processing performed by the frame memory correlation unit of the ultrasound observation apparatus according to an embodiment of the present invention.
FIG. 7 is a schematic diagram illustrating motion vector detection processing performed by the frame memory correlation unit of the ultrasound observation apparatus according to an embodiment of the present invention.
FIG. 8 is a schematic diagram illustrating motion vector detection processing performed by the frame memory correlation unit of the ultrasound observation apparatus according to an embodiment of the present invention.
FIG. 9 is a schematic diagram illustrating position correction processing performed by the frame memory position correction unit of the ultrasound observation apparatus according to an embodiment of the present invention.
FIG. 10 is a schematic diagram illustrating image combining processing performed by a weighting and adding unit of the ultrasound observation apparatus according to an embodiment of the present invention.

Description of Embodiments

Hereinafter, modes for carrying out the present invention (hereinafter, referred to as an embodiment or embodiments) will be described with reference to the attached drawings.

(Embodiments)

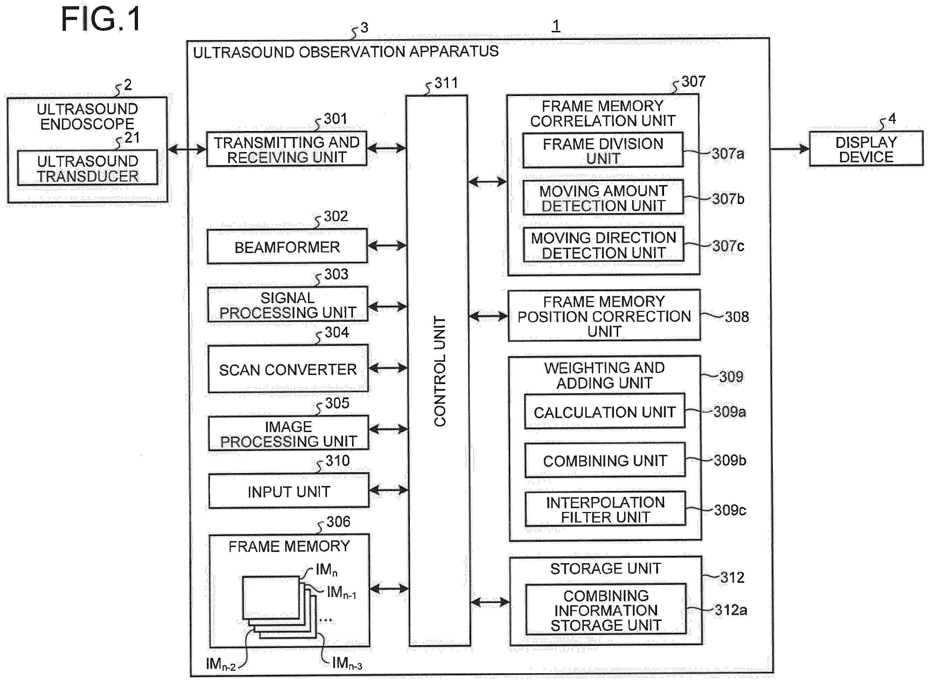

FIG. 1 is a block diagram illustrating a configuration of an ultrasound observation system including an ultrasound observation apparatus according to an embodiment of the present invention. An ultrasound diagnosis system 1 illustrated in FIG. 1 includes an ultrasound endoscope 2, an ultrasound observation apparatus 3, and a display device 4. The ultrasound endoscope 2 transmits ultrasound to a subject being an observation target and receives the ultrasound reflected on the subject. The ultrasound observation apparatus 3 generates an ultrasound image based on an ultrasound signal obtained by the ultrasound endoscope 2. The display device 4 displays the ultrasound image generated by the ultrasound observation apparatus 3.

The ultrasound endoscope 2 includes, on its distal end, an ultrasound transducer 21. The ultrasound transducer 21 converts an electrical pulse signal received from the ultrasound observation apparatus 3 into an ultrasound pulse (acoustic pulse) and emits the converted pulse to the subject. The ultrasound transducer 21 also converts an ultrasound echo reflected on the subject into an electrical echo signal expressed by a voltage change, and outputs the signal. The ultrasound transducer 21 may be any of a convex transducer, a linear transducer, and a radial transducer. The ultrasound endoscope 2 may cause the ultrasound transducer 21 to perform mechanical scan, or may provide, as the ultrasound transducer 21, a plurality of elements in an array, and may cause the ultrasound transducer to perform electronic scan by electronically switching elements related to transmission and reception or imposing delay onto transmission and reception in each element.

The ultrasound endoscope 2 typically includes imaging optics and imaging elements. The ultrasound endoscope 2 can be inserted into gastrointestinal tracts (esophagus, stomach, duodenum, and large intestine) or respiratory organs (trachea, bronchus) of the subject and can capture gastrointestinal tract, respiratory organs, and their surrounding organs (pancreas, gall bladder, bile duct, biliary tract, lymph nodes, mediastinal organs, blood vessels, or the like). The ultrasound endoscope 2 includes a light guide that guides illumination light emitted to the subject at the time of imaging. The light guide is configured such that a distal end portion thereof reaches a distal end of an insertion portion of the ultrasound endoscope 2 into the subject, while a proximal end portion thereof is connected to a light source apparatus that generates illumination light.

The ultrasound observation apparatus 3 includes a transmitting and receiving unit 301, a beamformer 302, a signal processing unit 303, a scan converter 304, an image processing unit 305, a frame memory 306, a frame memory correlation unit 307, a frame memory position correction unit 308, a weighting and adding unit 309, an input unit 310, a control unit 311 and a storage unit 312.

The transmitting and receiving unit 301 is electrically connected with the ultrasound endoscope 2, transmits a transmission signal (pulse signal) having a high-voltage pulse to the ultrasound transducer 21 based on a predetermined waveform and transmission timing, and receives an echo signal as an electrical reception signal, from the ultrasound transducer 21.

The frequency band of the pulse signal transmitted by the transmitting and receiving unit 301 is preferably a broadband substantially covering a linear response frequency band for electroacoustic conversion from pulse signals to ultrasound pulses on the ultrasound transducer 21.

The transmitting and receiving unit 301 has a function of transmitting various control signals output by the control unit 311, to the ultrasound endoscope 2, and has a function of receiving various types of information including ID from the ultrasound endoscope 2 and transmitting the information to the control unit 311.

The beamformer 302 receives an echo signal, from the transmitting and receiving unit 301, generates digital radio frequency (RF) signal data (hereinafter, referred to as RF data), and outputs the generated RF data. The beamformer 302 performs sensitivity time control (STC) correction that amplifies an echo signal having a larger reception depth by using a higher amplification factor, performs processing such as filtering on the amplified echo signal, and thereafter, generates RF data of time domain by performing A/D conversion on the signal, and outputs the RF data to the signal processing unit 303. In a case where the ultrasound endoscope 2 is configured to perform scanning electronically with the ultrasound transducer 21 having a plurality of elements arranged in array, the beamformer 302 includes a beam-combining multi-channel circuit corresponding to the plurality of elements.

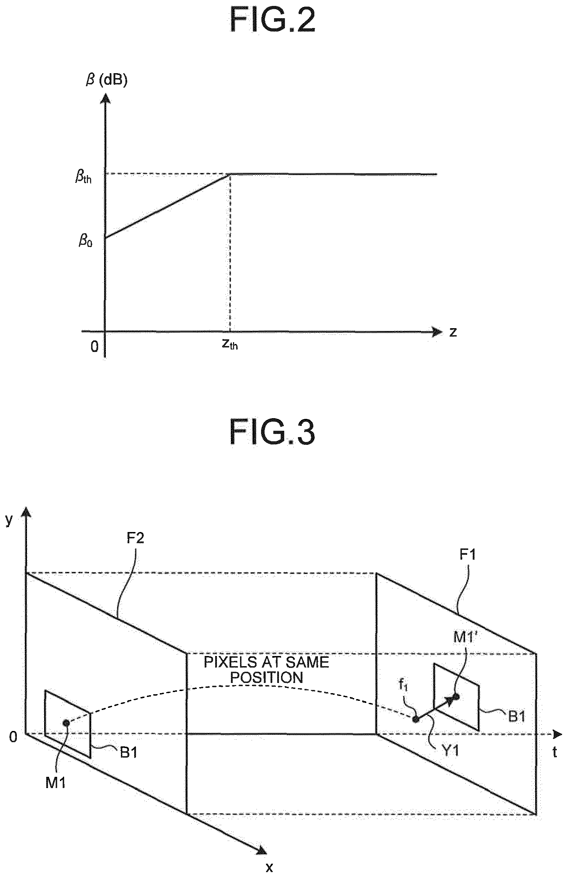

FIG. 2 is a diagram illustrating a relationship between a reception depth and an amplification factor in amplification processing performed by the beamformer 302. A reception depth z illustrated in FIG. 2 is an amount calculated based on elapsed time from a point of starting reception of the ultrasound. As illustrated in FIG. 2, in a case where the reception depth z is smaller than a threshold $z_{th}$, an amplification factor $\beta$ (dB) increases from $\beta_0$ to $\beta_{th}$ ($> \beta_0$) along with an increase in the reception depth z. In a case where the reception depth z is the threshold $z_{th}$ or above, the amplification factor $\beta$ (dB) takes a constant value $\beta_{th}$. The value of the threshold $z_{th}$ is a value at which an ultrasound signal received from the observation target is nearly completely attenuated and noise is dominant. More typically, in a case where the reception depth z is smaller than the threshold $z_{th}$, the amplification factor $\beta$ may preferably increase monotonically along with an increase in the reception depth z. The relationship illustrated in FIG. 2 is pre-stored in the storage unit 312.

The signal processing unit 303 generates digital reception data for B-mode based on the RF data received from the transmitting and receiving unit 301. The signal processing unit 303 performs known processing such as a band-pass filter, envelope detection, and logarithmic transformation, on the RF data, and generates the digital reception data for B-mode. In logarithmic transformation, a value is represented in a decibel value by dividing RF data by the reference voltage $V_c$ and then taking a common logarithm of this amount. The signal processing unit 303 outputs the generated reception data for B-mode to the image processing unit 305. The signal processing unit 303 is formed with a central processing unit (CPU), a variety of calculation circuits, or the like.

The scan converter 304 performs scan direction conversion on the reception data for B-mode received from the signal processing unit 303 and generates frame data. Specifically, the scan converter 304 converts the scan direction of the reception data for B-mode from the scan direction of the ultrasound to the display direction of the display device 4.

The image processing unit 305 generates B-mode image data (hereinafter, also referred to simply as image data) that includes a B-mode image as an ultrasound image displayed by converting amplitude of an echo signal into brightness. The image processing unit 305 performs signal processing on frame data from the scan converter 304 using known techniques such as gain processing and contrast processing, and generates B-mode image data by performing data decimation corresponding to a data step size defined in accordance with the display range of the image on the display device 4. The B-mode image is a gray-scale image on which values of R (red), G (green) and B (blue), namely, variables when the RGB color system is employed as a color space, match with each other.

The image processing unit 305 performs coordinate transformation on the reception data for B-mode from the signal processing unit 303 so as to rearrange a scanning range to be correctly represented in space, further fills gaps among individual reception data for B-mode by performing interpolation processing for individual reception data for B-mode, and generates B-mode image data.

The frame memory 306 is realized by a ring buffer, for example, and stores, in chronological order, a given amount (a predetermined number of frames N: N = n, n-1, n-2, n-3, ...) of B-mode images generated by the image processing unit 305. When the capacity is insufficient (when a predetermined number of frames of B-mode image data are stored), a predetermined number of frames of the latest B-mode images are stored in chronological order by overwriting the oldest B-mode image data with the latest B-mode image data. As illustrated in FIG. 1, the frame memory 306 stores a plurality of B-mode images ($IM_{n-1}$, $IM_{n-2}$, $IM_{n-3}$, ...) located at a predetermined number of frames back from the B-mode image $IM_n$, at the n-th frame (n is a natural number of two or more), that is the latest B-mode image.

The frame memory correlation unit 307 obtains correlation among the plurality of B-mode image data stored in the frame memory 306. Specifically, the frame memory correlation unit 307 obtains motion vector of a B-mode image (for example, B-mode images $IM_{n-1}$, $IM_{n-2}$, and $IM_{n-3}$) of the past frame based on the B-mode image (for example, B-mode image $IM_n$) of the latest frame. Then, the frame memory correlation unit 307 obtains three-dimensional correlation between the B-mode image of the frame that is latest (hereinafter, referred to as a latest frame) and the B-mode image of the frame of the past (hereinafter, referred to as a past frame), and thereafter, judges whether an object on an ultrasound image of the past frame has moved substantially in parallel on the image with respect to the object on an ultrasound image of the latest frame.

FIG. 3 is a diagram schematically illustrating motion between the images with different imaging timings performed by a frame memory correlation unit of the ultrasound observation apparatus according to an embodiment of the present invention. The frame memory correlation unit 307 generates a pseudo brightness signal by averaging R, G, and B for a first motion detection image F1 based on the B-mode image (B-mode image $IM_n$) of the latest frame and a second motion detection image F2 based on a B-mode image (B-mode image $IM_{n-1}$) of the past frame (e.g. the frame previous to the latest frame). As a method of motion detection, typical method of block matching processing is used for detection, for example. Specifically, detection is performed as to determine a position within the first motion detection image F1, to which a pixel M1 of the second motion detection image F2 has moved. The frame memory correlation unit 307 defines a block B1 (divided region) around a pixel M1 as a template, and scans a first motion detection image F1, using this template, namely, the block B1, around a pixel $f_1$ on the first motion detection image F1, that is at a position equal to the position of the pixel M1 on the second motion detection image F2. The frame memory correlation unit 307 then defines a center pixel of the position at which the sum-of-absolute-differences between the templates is the smallest as a pixel M1'. The frame memory correlation unit 307 detects, on the first motion detection image F1, a motion amount Y1 from the pixel M1 (pixel $f_1$) to the pixel M1' as a motion vector, and then, performs this processing on a central pixel of all divided regions as image processing targets. Hereinafter, coordinates of the pixel M1 will be defined as (x, y), and x components and y components of the motion vector on the coordinates (x, y) will be respectively described as Vx (x, y) and Vy (x, y). In the following, the x-direction corresponds to the right and left (horizontal) direction of the B-mode image, and the y-direction corresponds to the up-down (vertical) direction. Suppose that the coordinates of the pixel M1' on the first motion detection image F1 are defined as (x', y'), x' and y' are defined, respectively, as in the following equations (1) and (2).

$$x' = x + Vx(x, y) \quad ... \quad (1)$$

$$y' = y + Vy(x, y) \quad ... \quad (2)$$

The frame memory correlation unit 307 includes a frame division unit 307a, a moving amount detection unit 307b, and a moving direction detection unit 307c.

The frame division unit 307a divides a B-mode image so as to calculate the motion vector. Specifically, the B-mode image of the past frame as a correlation calculation target is divided into p × q (p and q represent natural numbers of two or above) and p × q divided regions are generated.

Based on the motion vector obtained by the frame memory correlation unit 307, the moving amount detection unit 307b calculates a moving amount for each of the divided regions generated by the frame division unit 307a. The moving amount detection unit 307b calculates an average moving amount for each of the frames based on the calculated moving amount. The moving amount detection unit 307b obtains a dispersion value (dispersion value L) of the moving amount (magnitude) as a decision value for deciding a moving state between the B-mode images based on the calculated moving amount. The moving amount detection unit 307b obtains, for example, dispersion values $L_{n-1}$, $L_{n-2}$, $L_{n-3}$, ... respectively for the B-mode image data of the n-1th or previous frames, for example.

Based on the motion vector obtained by the frame memory correlation unit 307, the moving direction detection unit 307c calculates a moving direction for each of the divided regions generated by the frame division unit 307a.

Based on the moving amount calculated by the moving amount detection unit 307b and based on the moving direction calculated by the moving direction detection unit 307c, the frame memory position correction unit 308 matches images between the frames by moving the B-mode image of the past frame with respect to the reference B-mode image $IM_n$.

The weighting and adding unit 309 determines a weighting amount of the B-mode image of the past frame to be combined with the reference B-mode image. Based on the determined weighting amount, the weighting and adding unit 309 performs combining processing on the B-mode image, then, performs interpolation processing on the combined image. The weighting and adding unit 309 includes a calculation unit 309a, a combining unit 309b, and an interpolation filter unit 309c.

Based on the average moving amount calculated by the moving amount detection unit 307b, the calculation unit 309a determines the weighting amount of the B-mode image of the past frame, to be combined with the reference B-mode image. The calculation unit 309a performs, for example, setting such that greater weighting amount is obtained for the B-mode images of the frame with a smaller average moving amount. The weighting amount is, for example, a coefficient to be multiplied with each of the converted brightness values.

Based on the weighting amount determined by the calculation unit 309a, the combining unit 309b combines the B-mode image of the reference frame with the B-mode image of the past frame. The combining unit 309b outputs the image after combining (hereinafter, referred to as a combined image) to the interpolation filter unit 309c.

Using a known space filter, the interpolation filter unit 309c corrects a discontinuous portion caused by misalignment of the images between the frames on the combined image. Specifically, by employing, as a space filter, a deconvolution filter, which performs blur correction by estimating blur at image combining using point spread function (PSF), the interpolation filter unit 309c corrects discontinuous portions of the image between the frames on the combined image, thereby suppressing blur on the combined image.

Alternatively, the interpolation filter unit 309c may use, as another type of space filter, a weighted average filter for calculating a pixel value between the pixels by performing weighting and averaging the pixel values of the pixels around the pixel of interest so as to correct discontinuous portion of the image between the frames on the combined image, and to suppress blur on the combined image.

The input unit 310 is formed with a user interface such as a keyboard, a mouse, and a touch panel, and receives input of various types of information.

The control unit 311 controls the entire ultrasound diagnosis system 1. The control unit 311 is formed with a central processing unit (CPU), a variety of calculation circuits, or the like, including calculation and control functions. The control unit 311 integrally controls the ultrasound observation apparatus 3 by reading from the storage unit 312, information stored in the storage unit 312, and by executing various types of calculation processing related to an operation method of the ultrasound observation apparatus 3. The control unit 311 can also be configured with a CPU, or the like, shared with the signal processing unit 303.

The storage unit 312 stores various types of information needed for operation of the ultrasound observation apparatus 3. The storage unit 312 includes a combining information storage unit 312a which stores a prescribed value α related to the dispersion value (dispersion value L) of the magnitude (moving direction) of the motion vector calculated by the frame memory correlation unit 307, a prescribed value γ related to the average moving amount (average moving amount D), and a weighting amount that is used for weighting processing and set according to the average moving amount.

Besides the above, the storage unit 312 also stores, for example, information needed for amplification processing (relationship between amplification factor and reception depth, illustrated in FIG. 2).

The storage unit 312 stores various programs including an operation program for executing an operation method of the ultrasound observation apparatus 3. The operation program can be recorded in a computer-readable recording medium such as a hard disk, flash memory, CD-ROM, DVD-ROM, or flexible disk, and can be distributed broadly. It is also possible to obtain the above-described various programs by downloading them via a communication network. Herein, the communication network refers to one implemented by, for example, a known public network, a local area network (LAN), or a wide area network (WAN), regardless of wired or wireless.

The storage unit 312 with the above-described configuration is implemented using a read only memory (ROM) in which various programs are pre-installed, a random access memory (RAM) storing calculation parameters and data for individual processing, and the like.

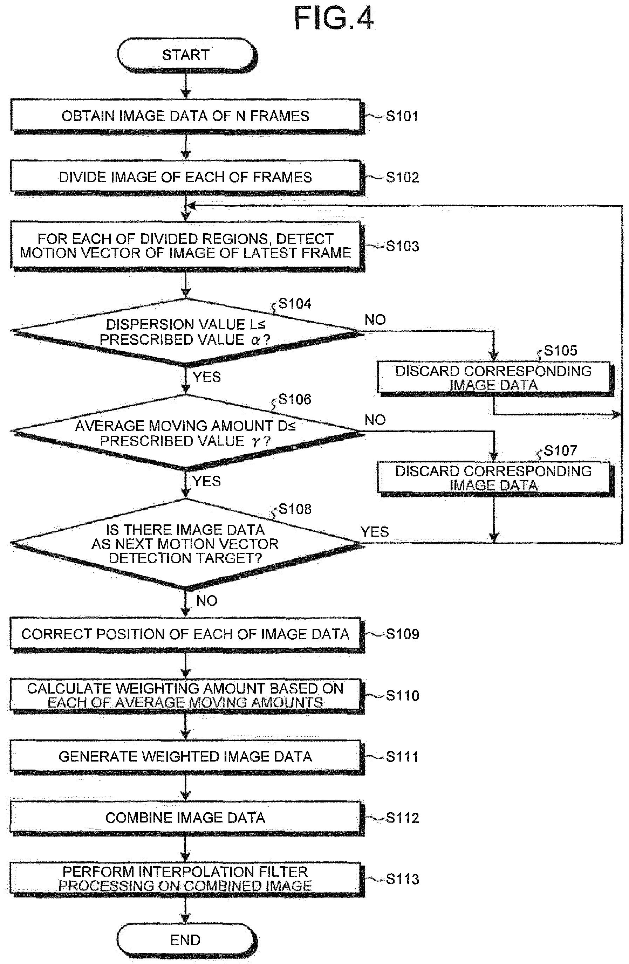

FIG. 4 is a flowchart illustrating an outline of the B-mode image combining processing performed by the ultrasound observation apparatus 3 having the above-described configuration. First, the frame memory correlation unit 307 obtains, from the frame memory 306, image data (B-mode image data) of N frames, including the B-mode image $IM_n$ of the latest frame (step S101).

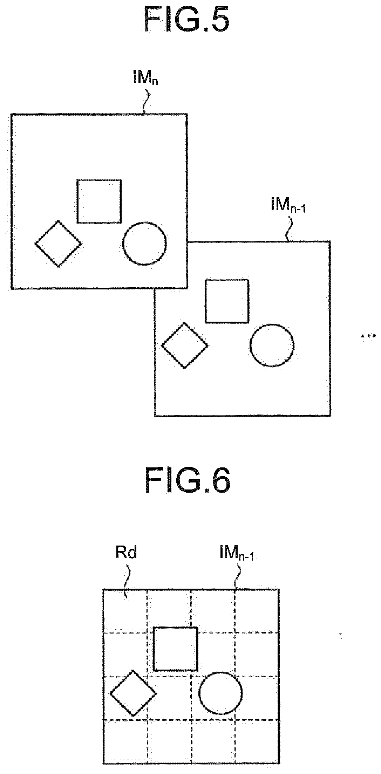

FIG. 5 is a schematic diagram illustrating motion vector detection processing performed by the frame memory correlation unit of the ultrasound observation apparatus according to an embodiment of the present invention. As illustrated in FIG. 5, the frame memory correlation unit 307 obtains image data (B-mode image data) of N frames, including the B-mode image $IM_n$ of the latest frame, stored in the frame memory 306.

After the image data of N frames have been obtained, the frame division unit 307a divides the B-mode image of each of the frames (past frames) excluding the B-mode images of the latest frame (step S102). FIG. 6 is a schematic diagram illustrating motion vector detection processing performed by the frame memory correlation unit of the ultrasound observation apparatus according to an embodiment of the present invention, and a diagram illustrating dividing processing by the frame division unit 307a. As illustrated in FIG. 6, the frame division unit 307a generates 16 divided regions Rd by dividing, for example, the n-1th B-mode image $IM_{n-1}$ into $4 \times 4$ regions.

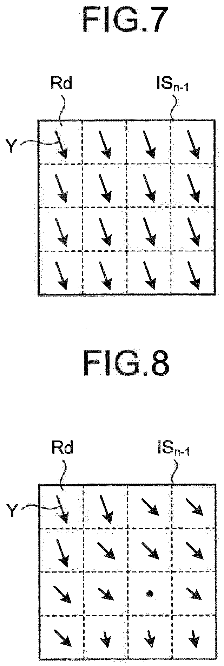

After the divided regions Rd have been generated, the frame memory correlation unit 307 detects, for each of the divided regions Rd, a motion vector with respect to the B-mode image $IM_n$ of the latest frame (reference frame) (step S103). FIG. 7 is a schematic diagram illustrating motion vector detection processing performed by the frame memory correlation unit of the ultrasound observation apparatus according to an embodiment of the present invention. The frame memory correlation unit 307 generates, for each of the frames, detected information associating the divided region Rd with a detected motion vector Y. For example, detected information $IS_{n-1}$ illustrated in FIG. 7 represents detected information on the n-1th frame.

After the motion vector is detected, the moving amount detection unit 307b calculates the moving amount for each of the divided regions Rd generated by the frame division unit 307a based on the detected information. Based on the calculated moving amount, the moving amount detection unit 307b obtains the dispersion value L (or a dispersion value $L_{n-1}$ for the n-1th frame, for example) of the corresponding frame.

Thereafter, the frame memory correlation unit 307 judges whether the obtained dispersion value L is the prescribed value $\alpha$ or below (step S104). The frame memory correlation unit 307 judges, for example, whether a dispersion value $L_{n-1}$ for the n-1th frame is the prescribed value $\alpha$ or below. In a case where the dispersion value $L_{n-1}$ is larger than the prescribed value $\alpha$ (step S104: No), the frame memory correlation unit 307 judges that there is large variance between the divided regions Rd in the moving direction, namely, the object movement state on the B-mode image is a movement having low correlation with the past frame, proceeds to step S105, and excludes (discards) the B-mode image with the corresponding frame number from combining. Thereafter, the frame memory correlation unit 307 repeats processing of steps S103 and S104 on a B-mode image of a frame as a next motion vector detection target.

In contrast, in a case where the dispersion value $L_{n-1}$ is the prescribed value $\alpha$ or below (step S104: Yes), the frame memory correlation unit 307 judges that there is small variance between the divided regions Rd in a three-dimensional moving direction, namely, the object movement state on the B-mode image is a movement having high correlation with the past frame, and proceeds to step S106.

In step S106, based on the motion vector obtained by the frame memory correlation unit 307, the moving amount detection unit 307b calculates a moving amount for each of the divided regions Rd, and then, calculates average moving amounts D ($D_{n-1}$, $D_{n-2}$, $D_{n-3}$, ...) based on the calculated moving amounts.

Thereafter, the frame memory correlation unit 307 judges whether the obtained average moving amount D is the prescribed value $\gamma$ or below (step S106). The frame memory correlation unit 307 judges, for example, whether an average moving amount $D_{n-1}$ for the n-1th frame is the prescribed value $\gamma$ or below. In a case where the average moving amount $D_{n-1}$ is larger than the prescribed value $\gamma$ (step S106: No), a moving amount between the divided regions Rd is large, and the region to be combined is small with respect to the reference B-mode image. Accordingly, the frame memory correlation unit 307 proceeds to step S107, and excludes (discards) the B-mode image of the corresponding frame number from the combining. Thereafter, the frame memory correlation unit 307 repeats processing of steps S103 and S104 on a B-mode image of a frame as a next motion vector detection target.

Although FIG. 7 illustrates the divided regions Rd having a same motion vector direction and size, there may be cases where, as illustrated in FIG. 8, the divided regions Rd have their own motion vector directions and sizes. FIG. 8 is a schematic diagram illustrating motion vector detection processing performed by the frame memory correlation unit of the ultrasound observation apparatus according to an embodiment of the present invention. On the detected information $IS_{n-1}$ illustrated in FIG. 8, the divided regions Rd have their own motion vector directions and sizes. In this case, the B-mode image of the corresponding frame number will be excluded (discarded) from the combining at the above-described step S105 or S107.

In a case where the average moving amount $D_{n-1}$ is the prescribed value $\gamma$ or below (step S106: Yes), the frame memory correlation unit 307 judges that the moving amount between the divided regions Rd is small and the region to be combined is large, and proceeds to step S108. In step S108, the frame memory correlation unit 307 judges whether there is a B-mode image as a next motion vector detection target. In a case where there is a B-mode image as the next motion vector detection target (step S108: Yes), the frame memory correlation unit 307 repeats processing of steps S103 and S104. In contrast, in a case where there is no B-mode image as the next motion vector detection target (step S108: No), the frame memory correlation unit 307 proceeds to step S109.

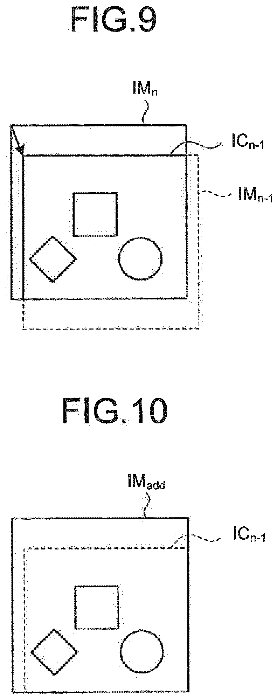

In step S109, based on the moving amount calculated by the moving amount detection unit 307b and based on the moving direction calculated by the moving direction detection unit 307c, the frame memory position correction unit 308 moves a relative position of the B-mode image of the past frame with respect to the reference B-mode image $IM_n$. FIG. 9 is a schematic diagram illustrating position correction processing performed by the frame memory correlation unit of the ultrasound observation apparatus according to an embodiment of the present invention. As illustrated in FIG. 9, the frame memory position correction unit 308 moves the B-mode image (B-mode image $IM_{n-1}$ in FIG. 9) of the past frame with respect to the B-mode image $IM_n$ of the reference frame. The frame memory position correction unit 308 segments a region (region to be combined) on the B-mode image $IM_{n-1}$, that overlaps with the B-mode image $IM_n$, and generates a segmented image $IC_{n-1}$ for combining.

Thereafter, based on the average moving amount, the calculation unit 309a determines the weighting amount of the B-mode image of the past frame, to be combined with the reference B-mode image (step S110). Alternatively, processing in steps S109 and S110 may be performed in the reverse order or concurrently.

After the weighting amount is determined, the combining unit 309b performs, based on the determined weighting amount, weighting processing on the B-mode image (segmented image for combining) of the past frame, generates weighted image data (step S111), and combines the B-mode image of the reference frame with the weighted image data of the past frame (step S112). FIG. 10 is a schematic diagram illustrating image combining processing performed by the weighting and adding unit of the ultrasound observation apparatus according to an embodiment of the present invention. As illustrated in FIG. 10, the combining unit 309b obtains a combined image $IM_{add}$ by combining the B-mode image $IM_n$ with the segmented image $IC_{n-1}$ for combining. Similarly, image data (segmented image for combining) of other past frames are combined. The combining unit 309b outputs the combined image that has undergone combining processing, to the interpolation filter unit 309c.

After the combined image is generated by the combining unit 309b, the interpolation filter unit 309c corrects a discontinuous portion, caused by misalignment of the images, between the frames on the combined image, by performing interpolation filter processing using a known space filter (step S113).

Under control of the control unit 311, the combined image generated by the above-described image combining processing is stored in the storage unit 312 and displayed on the display device 4. When a new echo signal is received from the ultrasound endoscope 2, the B-mode image of the latest frame in the frame memory 306 is updated. The ultrasound observation apparatus 3 performs the above-described image combining processing based on the latest B-mode image that has been updated.

According to an embodiment of the present invention described as above, the frame memory correlation unit 307 detects a moving amount and a moving direction, as three-dimensional correlation, of the ultrasound image of the past frame with respect to the ultrasound image of the latest frame, the frame memory position correction unit 308 moves the relative position of the ultrasound image of the past frame with respect to the ultrasound image of the latest frame based on the three-dimensional moving amount and moving direction that have been detected, and the weighting and adding unit 309 performs weighting processing on the ultrasound image of the past frame based on the three-dimensional moving amount and moving direction detected by the frame memory correlation unit 307. With this configuration, it is possible to achieve both noise reduction and blur reduction on the ultrasound image.

Embodiments of the present invention have been described hereinabove, however, the present invention is not intended to be limited to the above-described embodiments. For example, a living tissue has been employed as the observation target in the above-described embodiments. However, it is possible to apply the technique also to an industrial endoscope for observing material characteristics. The ultrasound observation apparatus according to the present invention can be applied to both inside and outside the body. Alternatively, it is possible to transmit and receive signals for observation target irradiated with infrared, or the like, in place of ultrasound.

In the above-described embodiments, an ultrasound image is divided to generate a plurality of divided regions, and a three-dimensional correlation between the ultrasound image of the past frame and the ultrasound image of the latest frame is obtained for each of the divided regions. Alternatively, it is also possible, for an entire ultrasound image, to obtain a three-dimensional correlation between the ultrasound image of the past frame and the ultrasound image of the latest frame, without dividing the entire ultrasound image. In this case, it would be merely desired to detect a feature point on the ultrasound image and obtain three-dimensional correlation regarding the detected feature point.

In this manner, the present invention may include various forms of embodiments without deviating from the technical spirit and scope of the general inventive concept as defined in the appended claims of this invention.

Industrial Applicability

The ultrasound observation apparatus according to the present invention is useful for achieving both noise reduction and blur reduction in an ultrasound image. Reference Signs List

| 1 | ULTRASOUND DIAGNOSIS SYSTEM |
| 2 | ULTRASOUND ENDOSCOPE |
| 3 | ULTRASOUND OBSERVATION APPARATUS |
| 4 | DISPLAY DEVICE |
| 21 | ULTRASOUND TRANSDUCER |
| 301 | TRANSMITTING AND RECEIVING UNIT |
| 302 | BEAMFORMER |
| 303 | SIGNAL PROCESSING UNIT |
| 304 | SCAN CONVERTER |
| 305 | IMAGE PROCESSING UNIT |
| 306 | FRAME MEMORY |
| 307 | FRAME MEMORY CORRELATION UNIT |
| 307a | FRAME DIVISION UNIT |
| 307b | MOVING AMOUNT DETECTION UNIT |
| 307c | MOVING DIRECTION DETECTION UNIT |
| 308 | FRAME MEMORY POSITION CORRECTION UNIT |
| 309 | WEIGHTING AND ADDING UNIT |
| 309a | CALCULATION UNIT |
| 309b | COMBINING UNIT |
| 309c | INTERPOLATION FILTER UNIT |
| 310 | INPUT UNIT |
| 311 | CONTROL UNIT |
| 312 | STORAGE UNIT |
| 312a | COMBINING INFORMATION STORAGE UNIT |

**Claims**

1. An ultrasound observation apparatus configured to generate a combined image by combining a plurality of ultrasound images generated based on an echo signal, the echo signal being obtained by converting an ultrasound echo into an electrical signal, the ultrasound echo being obtained by transmitting ultrasound to an observation target and by reflecting the ultrasound from the observation target, the ultrasound observation apparatus comprising:

    a frame memory configured to store the plurality of ultrasound images in chronological order;
a frame memory correlation unit configured to detect a moving amount and a moving direction as a three-dimensional correlation between an ultrasound image of a past frame and an ultrasound image of a latest frame, among the plurality of ultrasound images stored in the frame memory;
a frame memory position correction unit configured to move a relative position of the ultrasound image of the past frame with respect to the ultrasound image of the latest frame based on the three-dimensional moving amount and moving direction detected by the frame memory correlation unit; and
a weighting and adding unit configured to perform weighting processing on the ultrasound image of the past frame based on the three-dimensional moving amount detected by the frame memory correlation unit.

2. The ultrasound observation apparatus according to claim 1, wherein the weighting and adding unit is configured to perform the weighting processing on the ultrasound image of the past frame based on a weighting amount correlated with the moving amount detected by the frame memory correlation unit.

3. The ultrasound observation apparatus according to claim 1, wherein the frame memory correlation unit is configured to divide the ultrasound image of the past frame to generate divided regions, and to detect the three-dimensional moving amount and moving direction for each of the divided regions.

4. The ultrasound observation apparatus according to claim 3, wherein based on a plurality of three-dimensional moving amounts and moving directions each of which is detected for each of the divided regions, the frame memory correlation unit is configured to calculate the moving amount of whole of the ultrasound image of the past frame with respect to the ultrasound image of the latest frame.

5. The ultrasound observation apparatus according to claim 3, wherein
the frame memory correlation unit is configured to:

    calculate a representative moving amount of the ultrasound image of a current frame based on a plurality of three-dimensional moving amounts each of which is detected for each of the divided regions;
    compare the representative moving amount with a prescribed value; and
    exclude the ultrasound image of the current frame from combining according to a result of comparison.

6. The ultrasound observation apparatus according to claim 3, wherein based on a plurality of three-dimensional moving amounts and moving directions each of which is detected for each of the divided regions, the frame memory correlation unit is configured to identify a moving state of whole of the ultrasound image of the past frame with respect to the ultrasound image of the latest frame.

7. The ultrasound observation apparatus according to claim 3, wherein
the frame memory correlation unit is configured to:

   identify a moving state of the ultrasound image of a current frame based on a plurality of three-dimensional moving amounts each of which is detected for each of the divided regions; and
   exclude the ultrasound image of the current frame from combining, or reduce the weighting amount if the moving state differs from a moving state of the ultrasound image of the latest frame.

8. The ultrasound observation apparatus according to claim 6 or 7, wherein the moving state is a three-dimensional moving state of an object in the ultrasound images.

9. The ultrasound observation apparatus according to any one of claims 6 to 8, wherein the frame memory correlation unit is configured to identify the moving state by comparing a dispersion value of the moving amounts with a predetermined value.

10. The ultrasound observation apparatus according to claim 3, wherein the frame memory correlation unit is configured to detect a feature point from the ultrasound image of the latest frame, and to detect the correlation between the ultrasound image of the past frame and the ultrasound image of the latest frame by using the feature point.

# FIG.1

EP 3 235 437 A1

# FIG.2

# FIG.3

# FIG.4

```
                    ( START )
                        |
                        v
         OBTAIN IMAGE DATA OF N FRAMES          ~S101
                        |
                        v
         DIVIDE IMAGE OF EACH OF FRAMES         ~S102
                        |
                        v
      FOR EACH OF DIVIDED REGIONS, DETECT       ~S103
      MOTION VECTOR OF IMAGE OF LATEST FRAME
                        |
                        v
             DISPERSION VALUE L≤        NO
           PRESCRIBED VALUE α?    ----------->  DISCARD CORRESPONDING   ~S105
                  |  (S104)                         IMAGE DATA
                  | YES
                  v
          AVERAGE MOVING AMOUNT D≤     NO
           PRESCRIBED VALUE γ?   ------------>  DISCARD CORRESPONDING   ~S107
                  |  (S106)                         IMAGE DATA
                  | YES
                  v
            IS THERE IMAGE DATA        YES
          AS NEXT MOTION VECTOR  ---------------->
            DETECTION TARGET?
                 | (S108)
                 | NO
                 v
      CORRECT POSITION OF EACH OF IMAGE DATA    ~S109
                 |
                 v
      CALCULATE WEIGHTING AMOUNT BASED ON       ~S110
        EACH OF AVERAGE MOVING AMOUNTS
                 |
                 v
         GENERATE WEIGHTED IMAGE DATA           ~S111
                 |
                 v
             COMBINE IMAGE DATA                 ~S112
                 |
                 v
       PERFORM INTERPOLATION FILTER             ~S113
       PROCESSING ON COMBINED IMAGE
                 |
                 v
               ( END )
```

# FIG.5

# FIG.6

# FIG.7

# FIG.8

# FIG.9

IM_n

IC_{n-1}

IM_{n-1}

# FIG.10

IM_{add}

IC_{n-1}

## INTERNATIONAL SEARCH REPORT

| International application No. |
|---|
| PCT/JP2015/078840 |

A. CLASSIFICATION OF SUBJECT MATTER
*A61B8/08*(2006.01)i

According to International Patent Classification (IPC) or to both national classification and IPC

B. FIELDS SEARCHED

Minimum documentation searched (classification system followed by classification symbols)
A61B8/00-8/15

Documentation searched other than minimum documentation to the extent that such documents are included in the fields searched

| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2014-144370 A (Hitachi Medical Corp.), 14 August 2014 (14.08.2014), paragraphs [0012], [0018], [0021]; fig. 1 to 3 (Family: none) | 1-10 |
| A | JP 2012-19873 A (GE Medical Systems Global Technology Co., L.L.C.), 02 February 2012 (02.02.2012), paragraph [0101] & US 2012/0016237 A1 paragraph [0122] | 1-10 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 24 December 2015 (24.12.15) | 12 January 2016 (12.01.16) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (July 2009)

**INTERNATIONAL SEARCH REPORT**

| International application No. |
|---|
| PCT/JP2015/078840 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2014-87507 A  (GE Medical Systems Global Technology Co., L.L.C.), 15 May 2014 (15.05.2014), paragraph [0011] & US 2014/0119610 A1 paragraph [0011] | 1-10 |

Form PCT/ISA/210 (continuation of second sheet) (July 2009)

**EP 3 235 437 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010125789 A1